# EUROPEAN PATENT APPLICATION

(11) **EP 4 393 437 A1**
(43) Date of publication of application: **03.07.2024**
(21) Application number: 23220736.5
(22) Date of filing: 29.12.2023
(51) Int. Cl.: A61B 18/14, A61B 5/00, A61B 18/00

(54) **SYSTEMS AND METHODS FOR COUPLING SEGMENTED SPINE STRUTS**

(30) Priority: 30.12.2022 US 202263477988 P; 17.11.2023 US 202318512645
(71) Applicant: BIOSENSE WEBSTER (ISRAEL) LTD., 2066717 Yokneam (IL)
(72) Inventor: KEYES, Joseph Thomas, Irvine, 92618 (US); OKARSKI, Kevin Mark, Irvine, 92618 (US); JENKINS, Nathaniel, Irvine, 92618 (US); HERRERA, Kevin Justin, Irvine, 92618 (US); KHAN, Ishan, Irvine, 92618 (US); BEECKLER, Christopher Thomas, Irvine, 92618 (US)
(74) Representative: Carpmaels & Ransford LLP

(57) **Abstract**

The disclosed technology includes a segmented spine comprising a first electrode, a first spine strut, and a second spine strut. The first spine strut comprises a first attachment point configured to couple with the first electrode. The second spine strut comprises a second attachment point configured to couple with the first electrode. The first spine strut and second spine strut can comprise of additional attachment points configured to engage with a distal retention hub or a tubular shaft. The attachment points can be configured to permit the plurality of spine struts to rotate around the respective attachment points.

## Description

### CROSS-REFERENCE TO RELATED APPLICATIONS

This application claims the benefit of prior filed U.S. Provisional Patent Application No. 63/477,988 filed on December 30, 2022, which is hereby incorporated by reference as set forth in full herein.

### FIELD

The present invention relates generally to medical devices, and in particular catheters with electrodes, and further relates to, but not exclusively, the coupling of a plurality of segmented spine struts to electrodes at attachment points.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another. Medical probes may utilize radiofrequency (RF) electrical energy to heat tissue. Some ablation approaches use irreversible electroporation (IRE) to ablate cardiac tissue using nonthermal ablation methods.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Furthermore, multiple linear spines are generally assembled together by attaching both ends of the linear spines to a tubular shaft (e.g., a pusher tube) to form a spherical basket. Due to the small size of the spines and the electrodes, however, adhering the electrodes to the spines and then forming a spherical basket from the multiple linear spines can be a difficult task, increasing the manufacturing time and cost and the chances that the electrode fails due to an improper bond or misalignment. What is needed, therefore, are devices and methods of forming an improved basket assembly that can help to reduce the time required for manufacturing the basket assembly and alternative basket assembly geometries in general.

### SUMMARY

Various embodiments of a medical probe and related methods are described and illustrated. The medical probe may include a tubular shaft and an expandable basket assembly. The tubular shaft can have a proximal end and a distal end. The tubular shaft extends along a longitudinal axis. The expandable basket assembly can be positioned proximate the distal end of the tubular shaft. The basket assembly can include a structure that includes spine sections and a central spine intersection, a loop retention hub, and one or more electrodes. The central spine intersection can be positioned on the longitudinal axis at a distal end of the basket assembly. Each spine section can have at least one end connected to the distal end of the tubular shaft. The loop retention hub can include a first portion and a second portion configured to mate with each other to retain a distal portion of each of the spine sections at the central spine intersection. The electrode(s) can be coupled to each of the spine sections. Each electrode can define a lumen through the electrode so that a spine section extends through the lumen of each of the one or more electrodes. The spine sections or segmented spine struts can be coupled to each of the electrode(s) at attachment points. The attachment points can permit the segmented spine struts to rotate around each respective attachment point.

### BRIEF DESCRIPTION OF THE DRAWINGS

FIG. 1 is a schematic pictorial illustration of a medical system including a medical probe whose distal end has a basket assembly with electrodes coupled together, in accordance with an embodiment of the present invention;
FIG. 2 is a perspective view of an expandable basket assembly, in accordance with an embodiment of the present invention;
FIG. 3 is a perspective view of an inverted expandable basket assembly, in accordance with an embodiment of the present invention;
FIG. 4 is a perspective view of an expandable basket assembly, in accordance with an embodiment of the present invention;
FIG. 5 is a perspective view of an inverted expandable basket assembly, in accordance with an embodiment of the present invention;
FIG. 6 is a side view of a basket assembly in a collapsed form, in accordance with embodiments of the present invention;
FIGs. 7A and 7B are exploded side views of a medical probe, in accordance with an embodiment of the present invention;
FIG. 8 is a perspective view of a medical probe in an expanded form, in accordance with an embodiment of the present invention;
FIGs. 9A and 9B are side views of a spine loop of a given medical device, in accordance with embodiments of the present invention;
FIGs. 10A and 10B are a side view of a loop retention hub, in accordance with an embodiment of the present invention;
FIGs. 11A, 11B, and 11C are a top-down view of various loop retention hub locking mechanisms, in accordance with an embodiment of the present invention;
FIG. 12A is a perspective view of a loop retention hub of a self-expanding basket assembly, in accordance with an embodiment of the present invention;
FIG. 12B is a perspective view of a loop retention hub of an actuated expanding basket assembly, in accordance with an embodiment of the present invention;
FIGs. 13A, 13B, 13C, 13D, 13E, 13F, 13G, 13H, 131, and 13J are perspective views of various example electrodes, in accordance with embodiments of the present invention; and
FIG. 14 is a flowchart illustrating a method of assembling a basket assembly, in accordance with an embodiment of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode including a high current density and high electric flux density is positioned at a treatment site, and a second electrode including comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal including a positive-voltage phase pulse (referred to herein as "positive phase") and a negative-voltage phase pulse (referred to herein as "negative phase"). "Monophasic pulse" refers to an electrical signal including only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape including an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

The present disclosure is related to systems, methods or uses and devices which utilize segmented spines comprising electrodes coupled to spine struts at attachment points. Example systems, methods, and devices of the present disclosure may be particularly suited for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by programmed cell death or apoptosis, which is believed to leave less scar tissue as compared to other ablation modalities. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

Manufacturing and constructing of a medical probe capable of IRE as discussed above will now be discussed. Basket assemblies of medical probes can have a traumatic shape, particularly when it is expanded. Additionally, cutting the basket and current electrode installation can also be complicated. A solution is needed to overcome these installation issues that would also permit the formation of a basket assembly in various sizes and in multiple shapes.

The solution of this disclosure includes systems and methods for constructing a basket assembly. By coupling a plurality of spine struts together with an electrode at attachment points, manufacturing and installation issues as presented above can be avoided. A basket assembly that can easily be coupled together with attachment points permits the formation of basket assemblies of various sizes with spine struts of different sizes being coupled together. Attachment points also allow the spine struts to rotate about the attachment point to form various shapes with the plurality of spines.

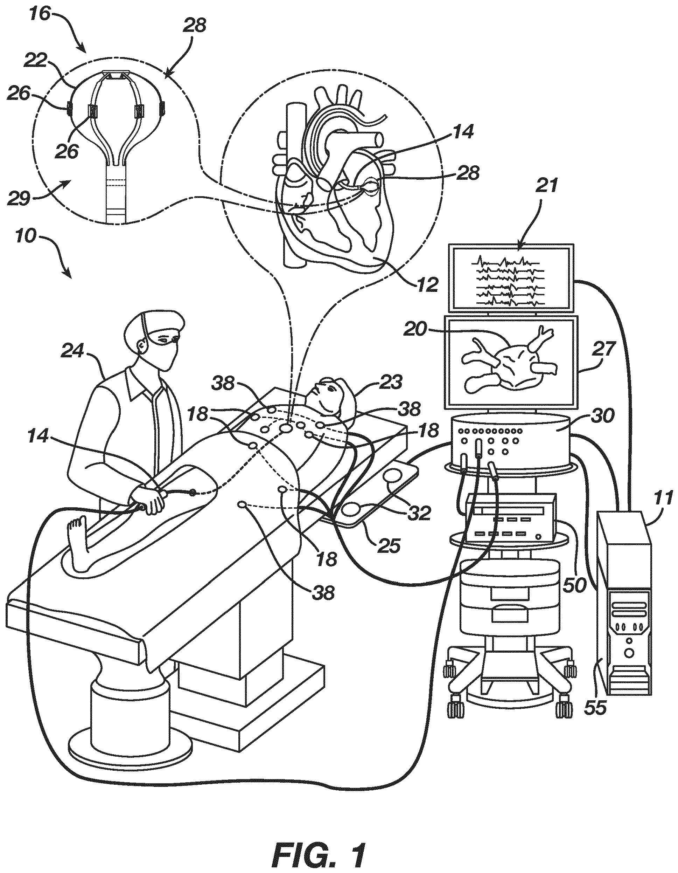

FIG. 1 is a schematic, pictorial illustration of a medical system 10 including a medical probe 16 and a patient interface unit 30, in accordance with an embodiment of the present invention. Medical system 10 may be based, for example, on the CARTO^{®} system, produced by Biosense Webster Inc. of 31 Technology Drive, Suite 200, Irvine, CA 92618 USA. In embodiments described hereinbelow, medical probe 16 can be used for diagnostic or therapeutic treatment, such as for performing ablation procedures in a heart 12 of a patient 23. Alternatively, medical probe 16 may be used, mutatis mutandis, for other therapeutic and/or diagnostic purposes in the heart or in other body organs.

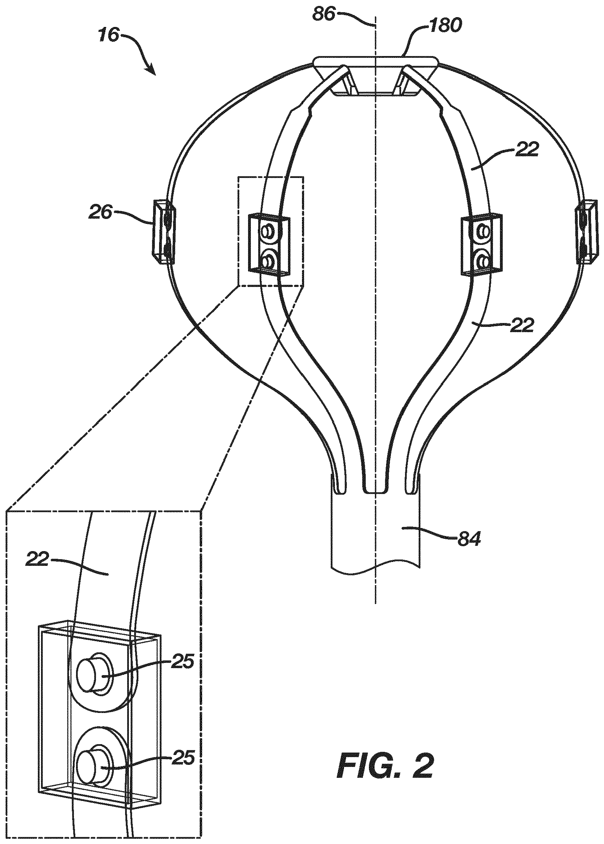

Medical probe 16 includes a plurality of segmented struts 22, electrodes 26 coupled to a proximal end of the tubular shaft. During a medical procedure, a medical professional 24 can insert the medical probe 16 through the vascular system of patient 23 so that a distal end 85 of the medical probe enters a body cavity such as a chamber of heart 12. Upon distal end 85 entering the chamber of heart 12, medical professional 24 can deploy a basket assembly 38 approximate a distal end 85 of the medical probe 16. Basket assembly 38 can include a plurality of electrodes 26 affixed to a plurality of segmented struts 22, as described in the description referencing FIGs. 2 through 6 hereinbelow. To start performing a medical procedure such as irreversible electroporation (IRE) ablation, medical professional 24 can manipulate a handle to position distal end 85 so that electrodes 26 engage cardiac tissue at a desired location or locations. Upon positioning the distal end 85 so that electrodes 26 engages cardiac tissue, the medical professional 24 can activate the medical probe 16 such that electrical pulses are delivered by the electrodes 26 to perform the IRE ablation.

The medical probe 16 can include a guide sheath and a therapeutic catheter, wherein the guide sheath includes the flexible insertion tube and the handle, and the therapeutic catheter includes the basket assembly 38, electrodes 26, and a tubular shaft 84 (see FIGs. 2 through 5). The therapeutic catheter is translated through the guide sheath so that the basket assembly 38 is positioned in the heart 12. The distal end 85 of the medical probe 16 corresponds to a distal end of the guide sheath when the basket assembly 38 is contained within the flexible insertion tube, and the distal end 85 of the medical probe 16 corresponds to a distal end of the basket assembly 38 when the basket assembly 38 is extended from the distal end of the guide sheath. The medical probe 16 can be alternatively configured to include a second handle on the therapeutic catheter and other features as understood by a person skilled in the pertinent art.

In the configuration shown in FIG. 1, patient interface unit 30 is connected, by a cable, to body surface electrodes, which typically include adhesive skin patches 44 that are affixed to patient 23. Patient interface unit 30 includes a processor that, in conjunction with a position sensor 29, determines location coordinates of distal end 85 inside heart 12. Location coordinates can be determined based on electromagnetic position sensor output signals provided from the distal portion of the catheter when in the presence of a generated magnetic field. Location coordinates can additionally, or alternatively be based on impedances and/or currents measured between adhesive skin patches and electrodes 26 that are affixed to basket assembly 38. In addition to being used as location sensors during a medical procedure, electrodes 26 may perform other tasks such as ablating tissue in the heart.

As described hereinabove, in conjunction with position sensor 29, processor may determine location coordinates of distal end 85 inside heart 12 based on impedances and/or currents measured between adhesive skin patches and electrodes 26. Such a determination is typically after a calibration process relating the impedances or currents to known locations of the distal end has been performed. While embodiments presented herein describe electrodes 26 that are preferably configured to deliver IRE ablation energy to tissue in heart 12, configuring electrodes 26 to deliver any other type of ablation energy to tissue in any body cavity is considered to be within the spirit and scope of the present invention. Furthermore, although described in the context of being electrodes 26 that are configured to deliver IRE ablation energy to tissue in the heart 12, one skilled in the art will appreciate that the disclosed technology can be applicable to electrodes used for mapping and/or determining various characteristics of an organ or other part of the patient's 23 body.

Processor may include real-time noise reduction circuitry typically configured as a field programmable gate array (FPGA), followed by an analog-to-digital (A/D) signal conversion integrated circuit 52. The processor can be programmed to perform one or more algorithms and uses circuitry and circuit as well as features of modules to enable the medical professional 24 to perform the IRE ablation procedure.

Patient interface unit 30 also includes an input/output (I/O) communications interface that enables patient interface unit 30 to transfer signals from, and/or transfer signals to electrodes 26 and adhesive skin patches. In the configuration shown in FIG. 1, patient interface unit 30 additionally includes an IRE ablation module and a switching module.

IRE ablation module is configured to generate IRE pulses including peak power in the range of tens of kilowatts. In some examples, the electrodes 26 are configured to deliver electrical pulses including a peak voltage of at least 700 volts (V). The medical system 10 performs IRE ablation by delivering IRE pulses to electrodes 26. Preferably, the medical system 10 delivers biphasic pulses between electrodes 26 on the spine. Additionally, or alternatively, the medical system 10 delivers monophasic pulses between at least one of the electrodes 26 and a skin patch.

Irrigation is sometimes utilized to reduce clot formation, stagnant blood flow or even reduce heat generated by ablation via the electrodes. As such, system 10 may supply irrigation fluid (e.g., a saline solution) to distal end 85 and to the electrodes 26 via a channel (not shown) in tubular shaft 84 (see FIGs. 2 through 5). Additionally, or alternatively, irrigation fluid can be supplied through the flexible insertion tube. Patient interface unit 30 includes an irrigation module to monitor and control irrigation parameters, such as the pressure and the temperature of the irrigation fluid.

Based on signals received from electrodes 26 and/or adhesive skin patches, processor can generate an electroanatomical map 20 that shows the location of distal end 85 in the patient's body. During the procedure, processor can present map 20 to medical professional 24 on a display 27, and store data representing the electroanatomical map 20 in a memory. Memory may include any suitable volatile and/or non-volatile memory, such as random-access memory or a hard disk drive.

In some embodiments, medical professional 24 can manipulate map 20 using one or more input devices. In alternative embodiments, display 27 may include a touchscreen that can be configured to accept inputs from medical professional 24, in addition to presenting map 20.

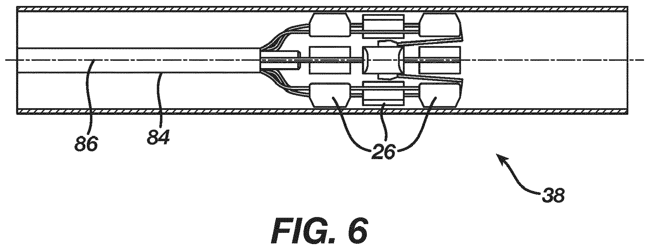

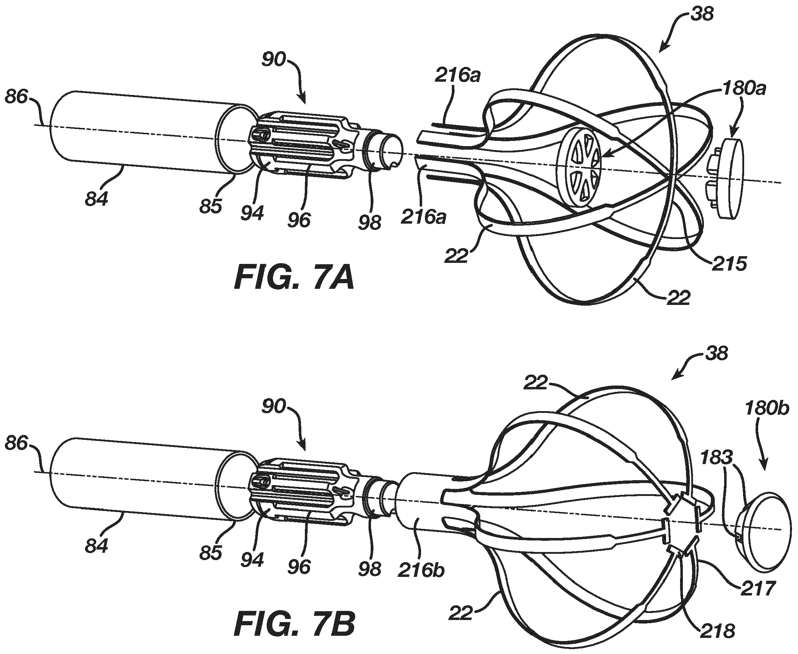

FIG. 2 is a perspective view of a medical probe 16 including a basket assembly 38 in an expanded form when unconstrained, such as by being advanced out of an insertion tube lumen (see FIG. 6) at a distal end 85 of an insertion tube. The medical probe 16 illustrated in FIG. 2 lacks the guide sheath illustrated in FIG. 1. The medical probe 16 includes segmented struts 22 (also known as spine sections) that are retained by a loop retention hub 180 at a distal end of the basket assembly 38. The segmented struts 22 include spine loops that have a distal loop 215 (see FIG. 7A) and two ends secured in the tubular shaft 84. The tubular shaft 84 is generally aligned along a longitudinal axis 86. The segmented spines 22 are coupled to electrodes 26 at attachment points 25. As illustrated in FIG. 2, a strut 22 can be coupled to the electrode 26 at an attachment point 25, and another strut 22 can be coupled to the same electrode 26 at another attachment point 25.

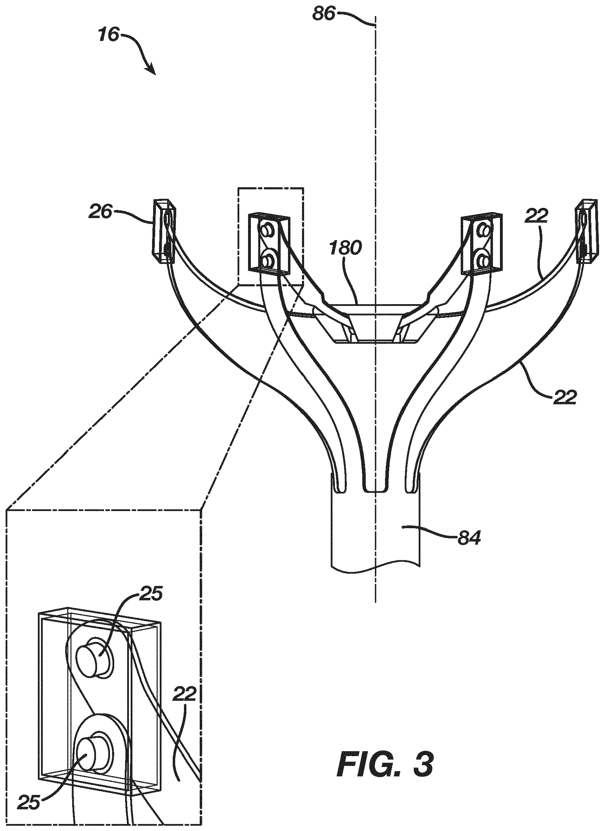

FIG. 3 shows a medical probe 16 including a basket assembly 38 in an expanded form when unconstrained, such as by being advanced out of an insertion tube lumen (see FIG. 6) at a distal end 85 of an insertion tube. The medical probe 16 illustrated in FIG. 2 lacks the guide sheath illustrated in FIG. 1. The medical probe 16 includes segmented struts 22 (also known as spine sections) that are retained by a loop retention hub 180 at a distal end of the basket assembly 38. The segmented struts 22 include spine loops that have a distal loop 215 (see FIG. 7A) and two ends secured in the tubular shaft 84. The tubular shaft 84 is generally aligned along a longitudinal axis 86. The segmented spines 22 are coupled to electrodes 26 at attachment points 25. As illustrated in FIG. 3, a strut 22 can be coupled to the electrode 26 at an attachment point 25, and another strut 22 can be coupled to the same electrode 26 at another attachment point 25. Further illustrated in FIG. 3, an attachment point 25 can allow a strut 22 to rotate about the attachment point 25 to create an inverted shape for the basket assembly 38.

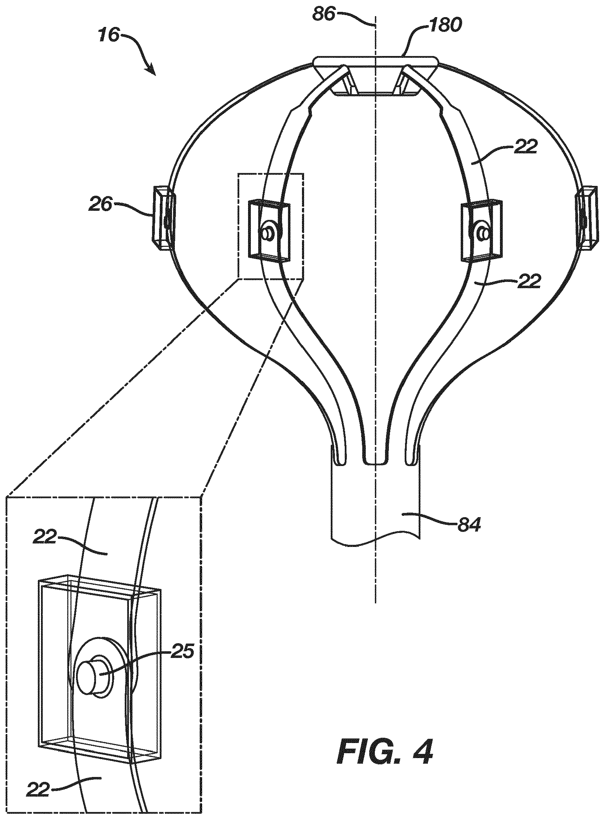

FIG. 4 illustrates a perspective view of a medical probe 16 including a basket assembly 38 in an expanded form when unconstrained, such as by being advanced out of an insertion tube lumen (see FIG. 6) at a distal end 85 of an insertion tube. The medical probe 16 illustrated in FIG. 2 lacks the guide sheath illustrated in FIG. 1. The medical probe 16 includes segmented struts 22 (also known as spine sections) that are retained by a loop retention hub 180 at a distal end of the basket assembly 38. The segmented struts 22 include spine loops that have a distal loop 215 (see FIG. 7A) and two ends secured in the tubular shaft 84. The tubular shaft 84 is generally aligned along a longitudinal axis 86. The segmented spines 22 are coupled to electrodes 26 at an attachment point 25. As illustrated in FIG. 4, a strut 22 can be coupled to the electrode 26 at the attachment point 25, and another strut 22 can be coupled to the same electrode 26 at the same attachment point 25.

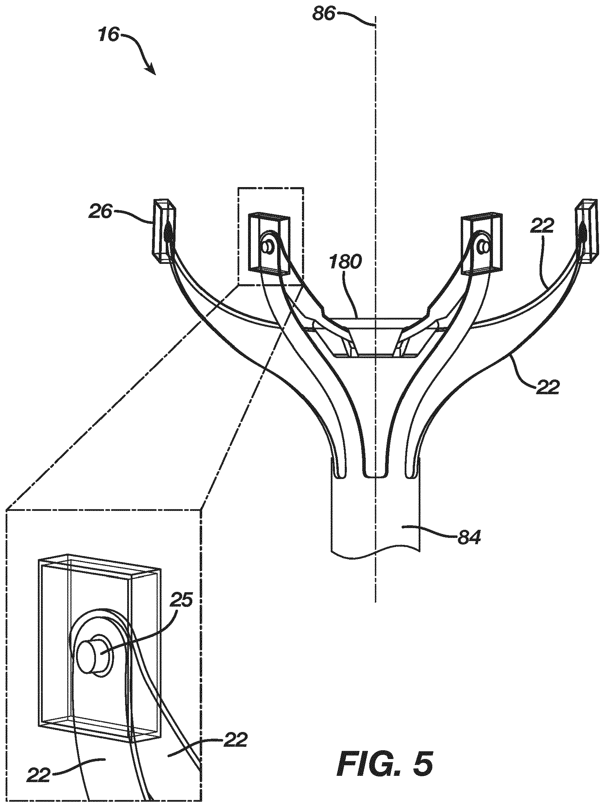

FIG. 5 is a perspective view of a medical probe 16 including a basket assembly 38 in an expanded form when unconstrained, such as by being advanced out of an insertion tube lumen (see FIG. 6) at a distal end 85 of an insertion tube. The medical probe 16 illustrated in FIG. 2 lacks the guide sheath illustrated in FIG. 1. The medical probe 16 includes segmented struts 22 (also known as spine sections) that are retained by a loop retention hub 180 at a distal end of the basket assembly 38. The segmented struts 22 include spine loops that have a distal loop 215 (see FIG. 7A) and two ends secured in the tubular shaft 84. The tubular shaft 84 is generally aligned along a longitudinal axis 86. The segmented spines 22 are coupled to electrodes 26 at an attachment point 25. As illustrated in FIG. 4, a strut 22 can be coupled to the electrode 26 at the attachment point 25, and another strut 22 can be coupled to the same electrode 26 at the same attachment point 25. Further illustrated in FIG. 5, an attachment point 25 can allow a strut 22 to rotate about the attachment point 25 to create an inverted shape for the basket assembly 38.

FIG. 6 shows a basket assembly 38 in a collapsed form that can be configured similar to the basket assembly 38 in FIGS. 2 through 5. The basket assembly 38 is collapsed within insertion tube of the guide sheath. In the expanded form (FIGs. 2 through 5), plurality of segmented struts 22 bow radially outwardly and in the collapsed form (FIG. 6) the segmented struts 22 are arranged generally along a longitudinal axis 86 of insertion tube.

Referring to FIGs. 2 through 6, during a medical procedure, medical professional 24 can deploy basket assembly 38 by extending tubular shaft 84 from insertion tube causing basket assembly 38 to exit insertion tube and transition to the expanded form. Spine struts 22 may have elliptical (e.g., circular) or rectangular (that may appear to be flat) cross-sections, and include a flexible, resilient material (e.g., a shape-memory alloy such as nickel-titanium, also known as Nitinol) forming a segmented strut 22 as will be described in greater detail herein.

In embodiments described herein, one or more electrodes 26 positioned on segmented struts 22 of basket assembly 38 can be configured to deliver ablation energy (RF and/or IRE) to tissue in heart 12. Additionally, or alternatively, the electrodes can also be used to determine the location of basket assembly 38 and/or to measure a physiological property such as local surface electrical potentials at respective locations on tissue in heart 12. The electrodes 26 can be biased such that a greater portion of the one or more electrodes 26 face outwardly from basket assembly 38 such that the one or more electrodes 26 deliver a greater amount of electrical energy outwardly away from the basket assembly 38 (i.e., toward the heart 12 tissue) than inwardly.

Examples of materials ideally suited for forming electrodes 26 include gold, platinum and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 12.

FIG. 7A is an exploded view of the medical probe 16 illustrated in FIG. 2. The electrodes 26 are omitted for the sake of illustration. The spine loops include distal loops 215 that overlap within the loop retention hub 180a. The spine loops include two ends that are secured between the tubular shaft 84 and relief lands 96 of a spine retention hub 90 that extends longitudinally from a distal end of the tubular shaft 84 towards the distal end of basket assembly 38.

FIG. 7B is an exploded view of the medical probe 16 illustrated in FIG. 2. The electrodes 26 are omitted for the sake of illustration. The spine sections 214b each include a stopper 218 that is retained within the loop retention hub 180b. The spine sections 214b each include a narrow distal portion 217 that can move longitudinally within slots 183 of the loop retention hub 180b when the stopper 218 rotates to expand or contract the basket assembly 38b. The stopper 218 can have extensions that extend orthogonal to the longitudinal axis to inhibit the stopper 218 from exiting the slots 183. The spine sections 214b extend distally from a proximal tube 216b. The proximal tube 216b and the spine sections 214b can be contiguous. In some embodiments, the spine sections 214b and proximal tube 216b can be cut from a singular tube. Alternatively, the spine sections 214b can have proximal ends.

Referring collectively to FIGs. 2 through 7B, the medical probe 16 can include a spine retention hub 90 disposed proximate the distal end 85 of the tubular shaft 84. The spine retention hub 90 can be inserted into the tubular shaft 84 and attached to the tubular shaft 84. Spine retention hub 90 can include a cylindrical member 94 including a plurality of relief lands 96, multiple irrigation openings 98, and at least one spine retention hub electrode. Relief lands 96 can be disposed on the outer surface of cylindrical member 94 and configured to allow a portion of each strut 22, such as each spine attachment end 216a, to be fitted into a respective relief land 96. The attachment end 216 can be a generally linear end of the spine. The attachment end 216 can be configured to extend outwardly from the spine retention hub 90 such that the basket assembly 38 is positioned outwardly from the spine retention hub 90 and, consequently, outwardly from the tubular shaft 84. In this way, the spine or segmented struts 22 can be configured to position the basket assembly 38 distally from the distal end of the tubular shaft 84 and distal from the distal end of the insertion tube when the basket assembly 38 is deployed. The relief lands 96 are preferably omitted when the medical probe 16 includes a proximal tube 216b joined to the spine sections.

As described supra, patient interface unit 30 includes irrigation module 60 that delivers irrigation fluid to distal end 85 of flexible insertion tube. The multiple irrigation openings 98 can be angled to spray or otherwise disperse of the irrigation fluid to either a given electrode 26 or to tissue in heart 12. Since electrodes 26 do not include irrigation openings 98 that deliver irrigation fluid, the configuration described hereinabove enables heat to be transferred from the tissue (i.e., during an ablation procedure) to the portion of the electrodes 26 on the inner side of the segmented struts 22b, and the electrodes 26 can be cooled by aiming the irrigation fluid, via irrigation openings 98, at the portion of the electrodes 26 on the inner side of the segmented struts 22b. Spine retention hub 90 electrode disposed at a distal end of retention hub 90 can be used in combination with electrodes 26 on the segmented struts 22, or alternatively, can be used independently from electrodes 26 for reference mapping or ablation.

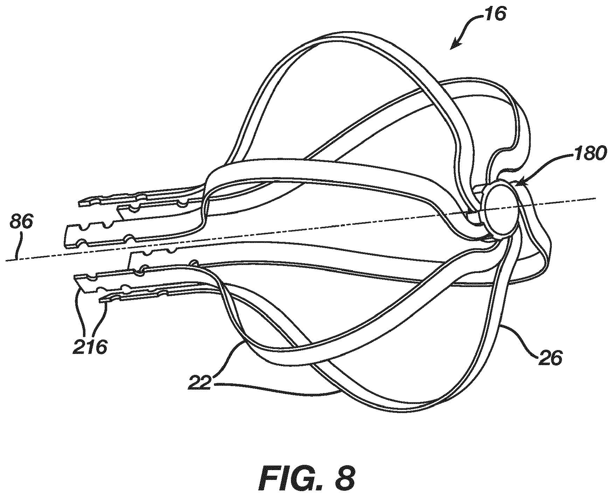

FIG. 8 illustrates a perspective view of an alternative configuration of spine sections 214c that have a curvature approximate the loop retention hub 180. As illustrated, the loop retention hub 180 can be configured similarly to the loop retention hub 180 illustrated in FIG. 2. Further, as illustrated, the spine sections 214 can include spine loops each including a distal loop 215 overlapped within the loop retention hub 180 and two ends that can be secured within the tubular shaft 84. Alternatively, the spine sections 214 illustrated in FIGs. 2 through 5 can be modified to include a curvature in the narrow distal portion 217 similar to the shape of the spine sections 214 illustrated in FIG. 8.

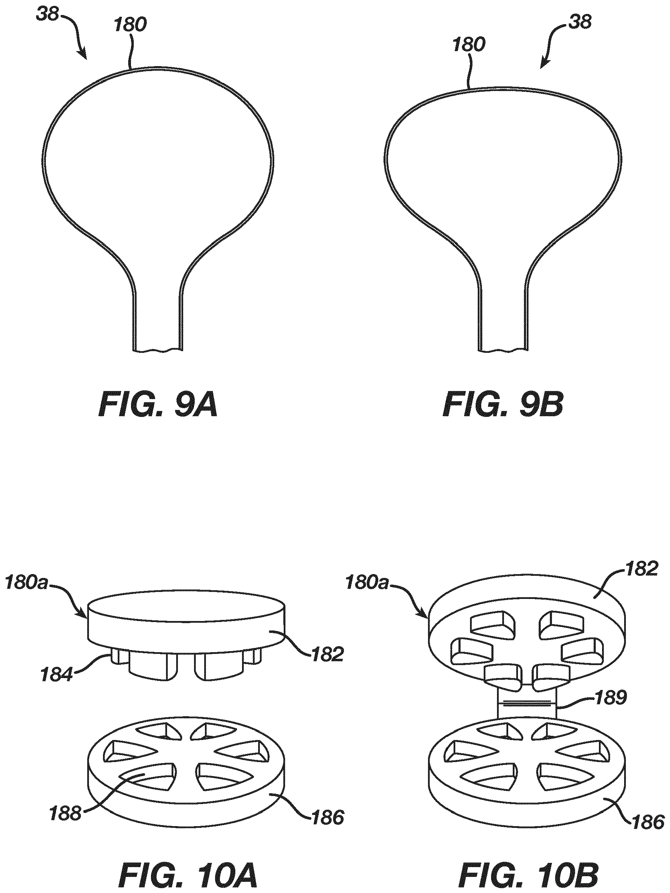

FIGs. 9A and 9B show a profile shape of a basket assembly 38 of a given medical device 22 when the spines sections 214 are expanded. As shown in FIG. 9A, the basket assembly 38 can be configured to form an approximately spheroid or spherical shape when in the expanded form. As another example, as shown in FIG. 9B, the basket assembly 38 can have an approximately elliptical profile and oblate-spheroid shape when in the expanded form. Although not every variation of shape is shown or described herein, one skilled in the art will appreciate that segmented struts 22 can be further configured to form other various shapes as would be suitable for the particular application.

By including segmented struts 22 configured to form various shapes when in the expanded form, basket assembly 38 can be configured to position the various electrodes 26 attached to segmented struts 22 at various locations, with each location being nearer or farther from the distal end of tubular shaft 84. For example, electrode 26 attached to spine 214 illustrated in FIG. 9A near the middle of spine 214 would be farther from the distal end of tubular shaft 84 than spine 214 illustrated in FIG. 9B when basket assembly 38 is in the expanded form. In addition, each spine 214 may have an elliptical (e.g., circular) or rectangular (that may appear to be flat) cross-section, and include a flexible, resilient material (e.g., a shape-memory alloy such as nickel-titanium (also known as Nitinol), cobalt chromium, or any other suitable material).

The examples above can have additional struts 22 and electrodes 26 to traverse from the insertion tube to the loop retention hub 180. Multiple struts 22 and electrodes 26 allow the basket assembly 38 to expand in size. In addition, the strut 22 can have common ends, i.e., attachment end 216, attachment point 25, and stoppers 218 (see below), to engage the various elements. A common strut 22 can be formed and continuously coupled together with electrodes 26 to form the basket assembly 38, minimizing production costs to increase the size of the basket assembly 38. The example ends can be identical on both the proximal and distal ends of the spine or a different one on each end, but matching to be modular to the attachment points.

FIGs. 10A and 10B show a side view of a loop retention hub 180a configured similarly to the loop retention hub 180a illustrated in FIGS. 2 through 6. The loop retention hub 180a includes a first portion 182 including protrusions 184 and a second portion 186 including indentations 188. The protrusions 184 engage the indentations 188 to clamp the first portion 182 to the second portion 186. The loop retention hub 180a can further include a hinge 189 between the first portion 182 and the second portion 186.

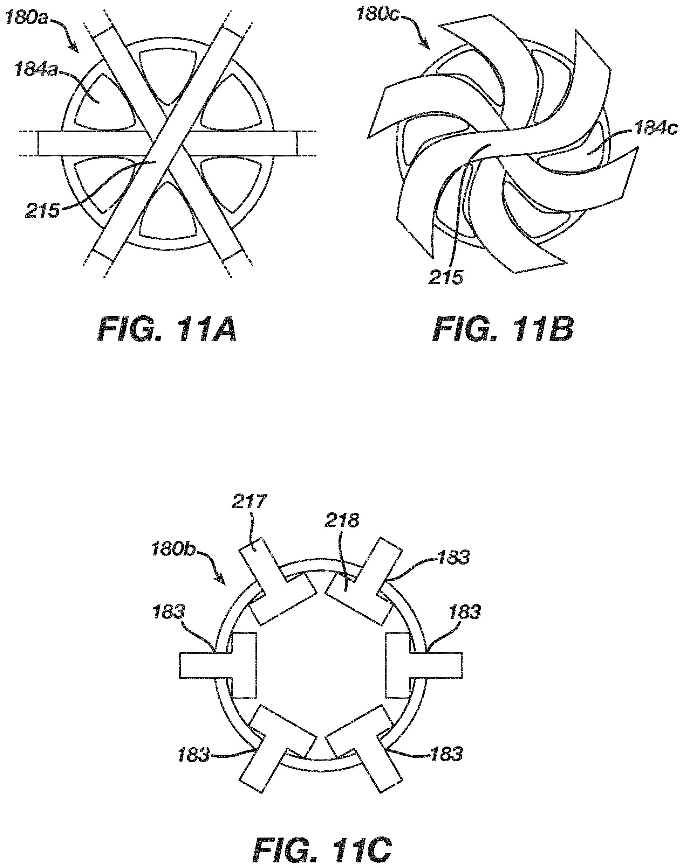

FIGs. 11A through 11C illustrate a top-down view of various loop retention hub locking mechanisms 184a, 184b, 184c.

FIG. 11A illustrates a loop retention hub 180a configured similarly to the loop retention hub 180a illustrated in FIGs. 10A and 10B. The loop retention hub 180a includes triangular protrusions 184a (and corresponding indentations not illustrated) defining linear paths between the protrusions 184a through which the distal loops 215 extend. The distal loops 215 overlap at a central spine intersection.

FIG. 11B illustrates a loop retention hub 180c configured similarly to the loop retention hub 180a illustrated in FIGs. 10A and 10B excepting that the protrusions 184c (and corresponding indentations not illustrated) include curvature to accommodate curves of distal loops 215 through paths between the protrusions 184c. The loop retention hub 180c may be particularly suited to retain spine sections 214c including curvature as illustrated in FIG. 8. The narrow distal portion 217 of each spine section 214b can extend through slots 183 of the loop retention hub 180c.

FIG. 11C illustrates a cross-section of the loop retention hub 180b illustrated in FIGS. 2 through 7. The stoppers 218 are positioned within the loop retention hub 180b and secured in loop retention hub 180b when clamped around the stoppers 218. The stoppers 218 are secured such that the spine sections 214 including narrow distal portion 217 can move longitudinally within slots 183 of the loop retention hub 180.

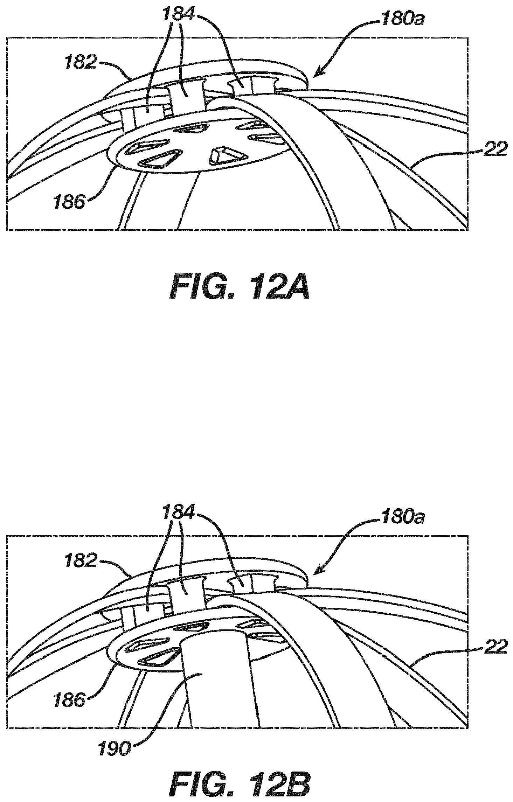

FIG. 12A is a perspective view of a loop retention hub 180a of a self-expanding basket assembly configured similarly to as illustrated in FIGs. 2, 7A, 10B, and 11A. The basket assembly 38 is configured to self-expand upon exiting a flexible insertion tube as described in relation to FIG. 6.

FIG. 12B is a perspective view of a loop retention hub 180a of an actuated expanding basket assembly 38. The basket assembly is configured similarly as illustrated in FIGs. 2, 7A, 10B, and 11A excepting that the medical probe 16 further includes a central member 190 movable along the longitudinal axis 86 in relation the tubular shaft 84 to expand and collapse the basket assembly 38. The central member 190 can include a distal end affixed to the second portion 186 of the loop retention hub 180a.

Referring collectively to FIGs. 2 through 12B, electrodes 26 can be attached to spine sections 214 before the spine sections are inserted into the tubular shaft 84 to form the basket assembly 38. As stated previously, the segmented struts 22 can include a flexible, resilient material (e.g., a shape-memory alloy such as nickel-titanium, also known as Nitinol) that can enable the basket assembly 38 to transition to its expanded form (as shown in FIG. 2) when the basket assembly 38 is deployed from flexible insertion tube. As will become apparent throughout this disclosure, segmented struts 22 can be electrically isolated from electrode 26 to prevent arcing from electrode 26 to the respective spine.

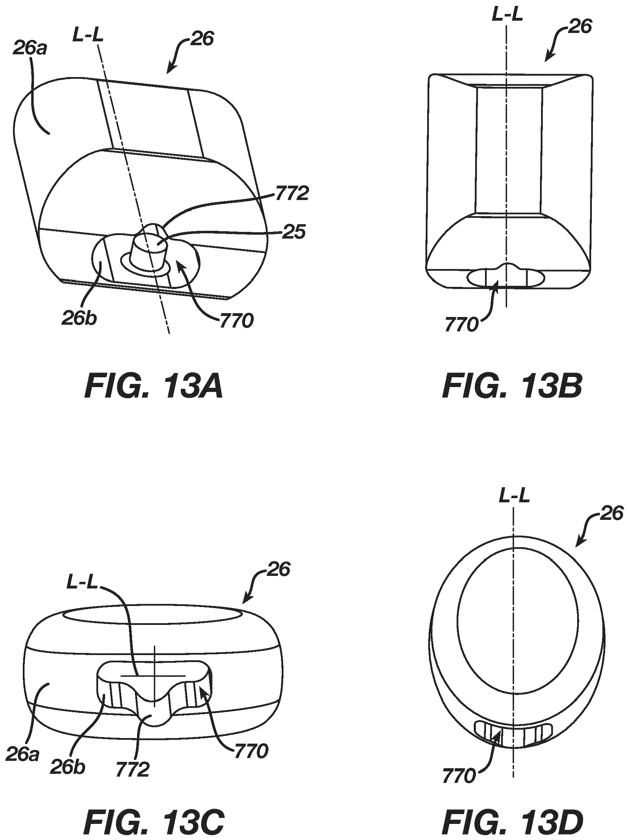

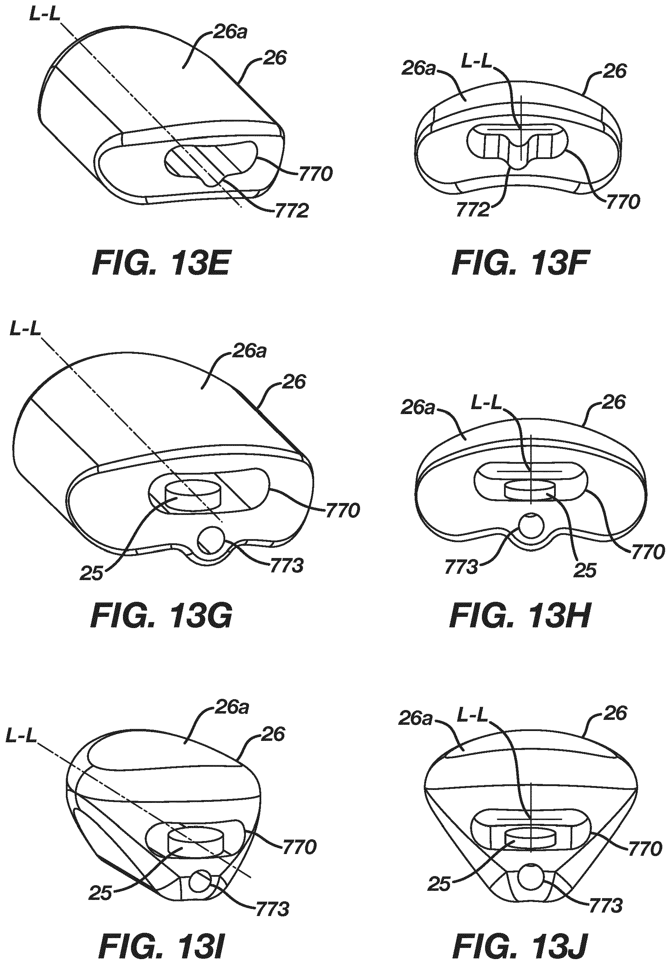

In some examples, each electrode 26 can include electrically conductive material (e.g., gold, platinum and palladium (and their respective alloys)). Turning to FIGs. 13A through 13J, electrode 26 can have a variety of cross-sectional shapes, curvatures, lengths, lumen number and lumen shape as provided as examples in electrodes 26a, 26b. The electrodes 26a, 26b are offered to illustrate various configurations of electrodes 26 that can be used with the medical device but should not be construed as limiting. One skilled in the art will appreciate that various other configurations of electrodes 26 can be used with the disclosed technology without departing from the scope of this disclosure.

Each electrode 26 can have an outer surface 26a facing outwardly from electrode 26 and an inner surface 26b facing inwardly toward electrode 26 where at least one lumen 770 is formed through electrode 26. The lumen 770 can be sized and configured to receive a spine such that spine can pass through electrode 26. Lumen 770 can be a symmetric opening through electrode 26a-26b and can be disposed offset with respect to a longitudinal axis L-L of the respective electrode. In other examples, lumen 770 can pass through electrode 26 in a generally transverse direction with respect to the longitudinal axis L-L of the respective electrode. Furthermore, lumen 770 can be positioned in electrode 26 nearer a bottom surface, nearer a top surface, or nearer a middle of electrode 26 depending on the particular configuration. In FIGs. 13A, 13C, and 13E through 13J, the top surface (upper side) is oriented toward the top of the drawing, the bottom surface (lower side) is oriented toward the bottom of the drawing, and the middle is between the top surface and the bottom surface. In other words, each electrode 26a-26b can include a lumen 770 that is offset with respect to a centroid of the electrode26a-26b.

In addition, as shown in FIGs. 13A through 13F, electrodes 26A-26C can have a wire relief 772 forming a recess or depression in electrode 26 adjacent lumen 770 for one or more wires to pass through lumen 770 along with a respective spine 214. Relief 772 can be sized to provide room for a wire of electrode 26 to pass through electrode 26 such that electrode 26 can be in electrical communication with the patient interface unit 30.

Alternatively, or in addition thereto, wires can pass through a wire lumen 773 as shown in example electrodes 26 in FIGs. 13G through 13J. Although not depicted, electrodes 26 may include both a wire relief 772 adjacent lumen 770 and wire lumen 773. Such electrode may permit additional wires to pass through the electrode body.

As shown in FIGs. 13A through 13J, the electrodes 26 can include various shapes depending on the application. For example, as illustrated in FIGs. 13A and 13B, the electrode 26 can comprise a substantially rectangular cuboid shape with rounded edges. In other examples, the electrode 26 can comprise a substantially ovoid shape (as illustrated in FIGs. 13C and 13D), the electrode 26 can have a contoured shape including a convex side and a concave side (as illustrated in FIGs. 13E through 13H), or the electrode 26 can have a contoured shape including substantially more material proximate an upper side than a lower side of the electrode 26 (as illustrated in FIGs. 13I and 13J). As will be appreciated by one of skill in the art, the various example electrodes 26 shown in FIGs. 13A through 13J, and described herein, are offered for illustrative purposes and should not be construed as limiting.

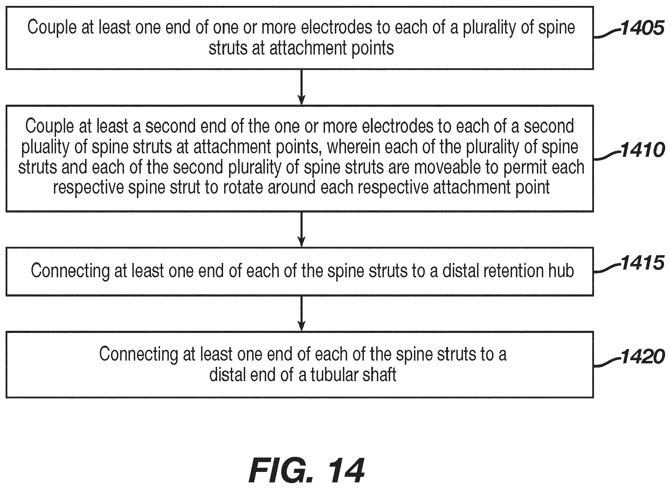

FIG. 14 is a flowchart illustrating a method 1400 of constructing a basket assembly 38, in accordance with an embodiment of the present invention. Method 1400 can include coupling (step 1405) at least one end of one or more electrodes 26 to each of a plurality of spine struts 222. The one or more electrodes 26 can be coupled to each of the spine struts 22 at attachment points. The method 1400 can include coupling (step 1410) at least a second end of the one or more electrodes 26 to each of a second plurality of spine struts 22. Each of the plurality of spine struts 22 and each of the second plurality of spine struts 22 can be moveable to permit each respective spine strut to rotate around each respective attachment point. Method 1400 can include connecting (step 1415) at least one end of each of the spine struts 22 to a distal retention hub. Method 1400 can include connecting (step 1420) at least one end of the spine struts 22 to a distal end of a tubular shaft 84.

As will be appreciated by one skilled in the art, method 1400 can include any of the various features of the disclosed technology described herein and can be varied depending on the particular configuration. Thus, method 1400 should not be construed as limited to the particular steps and order of steps explicitly described herein. It is noted that while the preference for the exemplary embodiments of the medical probe is for IRE or PFA, it is within the scope of the present invention to also use the medical probe separately only for RF ablation (unipolar mode with an external grounding electrode or bipolar mode) or in combination with IRE and RF ablations sequentially (certain electrodes in IRE mode and other electrodes in RF mode) or simultaneously (some electrodes in IRE mode and other electrodes in RF mode).

The disclosed technology described herein can be further understood according to the following clauses:
Clause 1: A segmented spine comprising: a first electrode; a first spine strut comprising a first attachment point configured to couple with the first electrode; and a second spine strut comprising a second attachment point configured to couple with the first electrode.
Clause 2: The segmented spine according to Clause 1, wherein the first spine strut further comprises a third attachment point configured to engage with a distal retention hub.
Clause 3: The segmented spine according to Clause 1, wherein the second spine strut further comprises a fourth attachment point configured to engage with a tubular shaft.
Clause 4: The segmented spine according to Clause 1, wherein the first attachment point of the first spine strut and the second attachment point of the second spine strut are configured to permit the first spine strut and second spine strut to rotate around the respective attachment point.
Clause 5: The segmented spine according to any of Clauses 1-4, further comprising: a second electrode; a third spine strut comprising a fifth attachment point configured to engage with the second electrode; and a fourth spine strut comprising a sixth attachment point configured to engage with the second electrode.
Clause 6: The segmented spine according to Clause 5, wherein the third spine strut further comprises a seventh attachment point configured to engage with a distal retention hub.
Clause 7: The segmented spine according to Clause 5, wherein the fourth spine strut further comprises an eighth attachment point configured to engage with a tubular shaft.
Clause 8: The segmented spine according to Clause 5, wherein the fifth attachment point of the third spine strut and the sixth attachment point of the fourth spine strut are configured to permit the third spine strut and fourth spine strut to rotate around the respective attachment point.
Clause 9: The segmented spine according to any of Clauses 1-8, wherein a plurality of segmented spines are configured to move from an inverted tubular configuration to an expanded spherical configuration.
Clause 10: An expandable basket assembly comprising: a plurality of segmented spines disposed about a longitudinal axis and coupled to each other, each of the plurality of segmented spines comprising: a first electrode disposed along the longitudinal axis; a first spine strut coupled to the first electrode; and a second spine strut coupled to the first electrode.
Clause 11: The expandable basket assembly according to Clause 10, wherein the first spine strut further comprises a second attachment point configured to engage with a distal retention hub.
Clause 12: The expandable basket assembly according to Clause 10, wherein the second spine strut further comprises a third attachment point configured to engage with a tubular shaft.
Clause 13: The expandable basket assembly according to any of Clauses 10-13, wherein a first attachment point of the first spine strut and the second spine strut are configured to permit the first spine strut and second spine strut to rotate around the first attachment point.
Clause 14: The expandable basket assembly according to any of Clauses 10-13, wherein the plurality of segmented spines are configured to move from an inverted tubular configuration to an expanded spherical configuration.
Clause 15: The expandable basket assembly according to any of Clauses 10-14, wherein each of the plurality of segmented spines further comprise: a second electrode; a third spine strut; and a fourth spine strut, wherein the third spine strut and the fourth spines strut each comprise a fourth attachment point configured to engage with the second electrode.
Clause 16: The expandable basket assembly according to Clause 15, wherein the third spine strut further comprises a fifth attachment point configured to engage with a distal retention hub.
Clause 17: The expandable basket assembly according to Clause 15, wherein the fourth spine strut further comprises a sixth attachment point configured to engage with a tubular shaft.
Clause 18: The expandable basket assembly according to any one of Clauses 10-17, wherein the plurality of segmented spines are configured to form geographical configurations.
Clause 19: The expandable basket assembly according to any one of Clauses 10-18, wherein the plurality of segmented spines are configured to form a first portion and a second portion configured to mate with each other to retain a distal portion of each of the plurality of segmented spines at a central spine intersection.
Clause 20: The expandable basket assembly according to any one of Clauses 10-19, wherein the expandable basket assembly defines a spherical outer profile.
Clause 21: The expandable basket assembly according to any one of Clauses 10-20, wherein the expandable basket assembly defines an oblate-spheroid profile.
Clause 22: The expandable basket assembly according to any one of Clauses 10-21, wherein each electrode is configured to deliver electrical pulses for irreversible electroporation, the electrical pulses including a peak voltage of at least 900 volts (V).
Clause 23: The expandable basket assembly according to any one of Clauses 10-22, further comprising a spine retention hub disposed proximate the distal end of a tubular shaft, the spine retention hub comprising a cylindrical member including a plurality of relief lands disposed on an outer surface of the cylindrical member to allow each spine strut to be fitted into the relief land and retained therein, the spine retention hub further comprising at least one electrode disposed at a distal portion of the spine retention hub.
Clause 24: The expandable basket assembly according to any one of Clauses 23, wherein the plurality of segmented spines comprises spine loops, each spine loop comprising a single unitary loop including a distal loop and two ends secured between the tubular shaft and in one of the relief lands of the spine retention hub.
Clause 25: The expandable basket assembly according to Clause 24, wherein the distal loops of each spine loop overlap within the distal retention hub.
Clause 26: The expandable basket assembly according to Clause 25, wherein the distal retention hub further comprises: two or more protrusions positioned on a first portion and/or a second portion; and two or more indentations positioned on the opposite portion of the first portion and the second portion, the indentations engaging the protrusions to clamp the first portion to the second portion.
Clause 27: The expandable basket assembly according to Clause 26, wherein the plurality of segmented spines fit within paths formed between the two or more protrusions.
Clause 28: The expandable basket assembly according to any one of Clauses 10-27, wherein each electrode comprises a wire relief adjacent a lumen to allow for one or more wires to extend adjacent to the lumen.
Clause 29: The expandable basket assembly according to Clause 28, wherein the lumen is disposed symmetrically about a longitudinal axis of each electrode.
Clause 30: The expandable basket assembly according to any one of Clauses 28-29, wherein the lumen is disposed offset with respect to a longitudinal axis of each electrode.
Clause 31: The expandable basket assembly according to any one of Clauses 10-30, further comprising a plurality of insulative sleeves each disposed over the respective given spine strut and within the lumen of the respective electrode.
Clause 32: The expandable basket assembly according to any one of Clauses 10-31, further comprising: a plurality of wires each electrically joined to a respective electrode, wherein at least a portion of the wires of the plurality of the wires respectively comprises an electrically conductive core material comprising a first electrical conductivity, an electrically conductive cover material comprising a second electrical conductivity less than the first electrical conductivity, the electrically conductive cover material circumscribing the electrically conductive core material, and an insulative jacket circumscribing the electrically conductive cover material.
Clause 33: The expandable basket assembly according to any one of Clauses 10-31, further comprising: a plurality of wires each electrically joined to a respective electrode, wherein at least a portion of the wires of the plurality of the wires respectively comprises a plurality of strands and an insulative jacket circumscribing the plurality of the strands, and wherein each strand of the plurality of strands respectively comprises an electrically conductive core material comprising a first electrical conductivity and an electrically conductive cover material comprising a second electrical conductivity less than the first electrical conductivity, the electrically conductive cover material circumscribing the electrically conductive core material.
Clause 34: The expandable basket assembly according to any one of Clauses 10-33, wherein the plurality of segmented spines comprises nitinol.
Clause 35: The expandable basket assembly according to any one of Clauses 10-33, wherein the plurality of segmented spines comprises metallic strands.
Clause 36: A method of constructing an expandable basket assembly, the method comprising: coupling at least one end of one or more electrodes to each of a plurality of spine struts, the one or more electrodes being coupled to each of the spine struts at attachment points; and coupling at least a second end of the one or more electrodes to each of a second plurality of spine struts, each of the plurality of spine struts and each of the second plurality of spine struts are moveable to permit each respective spine strut to rotate around each respective attachment point.
Clause 37: The method of Clause 36, further comprising: connecting at least one end of each of the spine struts to a distal retention hub.
Clause 38: The method of any one of Clauses 36-37, further comprising: connecting at least one end of each of the spine struts to a distal end of a tubular shaft.
Clause 39: The method of any one of Clauses 36-38, further comprising: configuring the plurality of spine struts and the second plurality of spine struts to extend radially outward from a longitudinal axis to define a shape.
Clause 40: The method of any one of Clauses 36-39, wherein the plurality of spine struts and the second plurality of spine struts are configured to move from an inverted tubular configuration to an expanded spherical configuration.
Clause 41: The method of any one of Clauses 39-40, wherein the shape is approximately spherical.
Clause 42: The method of any one of Clauses 39-41, wherein the shape is approximately oblate-spheroid.
Clause 43: The method of any one of Clauses 39-42, wherein the shape is a basket shape.
Clause 44: The method of Clause 43, wherein the basket shape is configured to be opened by disconnecting electrodes from spine struts at the attachment points.
Clause 45: The method of any one of Clauses 38-44, wherein the plurality of spine struts comprises spine loops, each spine loop comprising a single unitary loop including a distal loop, the method further comprising: securing two ends of each of the spine loops in the tubular shaft.
Clause 46: The method of any one of Clauses 37-45 further comprising: overlapping distal portions of each spine strut within the distal retention hub.
Clause 47: The method of any one of Clauses 37-46, further comprising: engaging two or more protrusions on a first portion and/or a second portion to two or more indentations on an opposite portion of the first portion and the second portion.
Clause 48: The method of any one of Clauses 37-47, further comprising: fitting the plurality of spine struts and the second plurality of spine struts within paths formed between the two or more protrusions.

The embodiments described above are cited by way of example, and the present invention is not limited by what has been particularly shown and described hereinabove. Rather, the scope of the invention includes both combinations and sub combinations of the various features described and illustrated hereinabove, as well as variations and modifications thereof which would occur to persons skilled in the art upon reading the foregoing description and which are not disclosed in the prior art.

## Claims

1. A segmented spine comprising:
a first electrode;
a first spine strut comprising a first attachment point configured to couple with the first electrode; and
a second spine strut comprising a second attachment point configured to couple with the first electrode.

2. The segmented spine according to claim 1, wherein the first spine strut further comprises a third attachment point configured to engage with a distal retention hub.

3. The segmented spine according to claim 1, wherein the second spine strut further comprises a fourth attachment point configured to engage with a tubular shaft.

4. The segmented spine according to claim 1, wherein the first attachment point of the first spine strut and the second attachment point of the second spine strut are configured to permit the first spine strut and second spine strut to rotate around the respective attachment point.

5. The segmented spine according to claim 1, further comprising:
a second electrode;
a third spine strut comprising a fifth attachment point configured to engage with the second electrode; and
a fourth spine strut comprising a sixth attachment point configured to engage with the second electrode.

6. The segmented spine according to claim 5, wherein (i) the third spine strut further comprises a seventh attachment point configured to engage with a distal retention hub, and the fourth spine strut further comprises an eighth attachment point configured to engage with a tubular shaft, or (ii) the fifth attachment point of the third spine strut and the sixth attachment point of the fourth spine strut are configured to permit the third spine strut and fourth spine strut to rotate around the respective attachment point.

7. The segmented spine according to claim 1, wherein a plurality of segmented spines are configured to move from an inverted tubular configuration to an expanded spherical configuration.

8. An expandable basket assembly comprising:
a plurality of segmented spines disposed about a longitudinal axis and coupled to each other, each of the plurality of segmented spines comprising:
a first electrode disposed along the longitudinal axis;
a first spine strut coupled to the first electrode; and
a second spine strut coupled to the first electrode.

9. The expandable basket assembly according to claim 8, wherein the first spine strut further comprises a second attachment point configured to engage with a distal retention hub, and the second spine strut further comprises a third attachment point configured to engage with a tubular shaft.

10. The expandable basket assembly according to claim 8, wherein a first attachment point of the first spine strut and the second spine strut are configured to permit the first spine strut and second spine strut to rotate around the first attachment point.

11. The expandable basket assembly according to claim 8, wherein the plurality of segmented spines are configured to move from an inverted tubular configuration to an expanded spherical configuration.

12. The expandable basket assembly according to claim 8, wherein each of the plurality of segmented spines further comprise:
a second electrode;
a third spine strut; and
a fourth spine strut,
wherein the third spine strut and the fourth spines strut each comprise a fourth attachment point configured to engage with the second electrode, optionally wherein the third spine strut further comprises a fifth attachment point configured to engage with a distal retention hub,
and the fourth spine strut further comprises a sixth attachment point configured to engage with a tubular shaft.

13. The expandable basket assembly according to claim 8, wherein the plurality of segmented spines are configured to form a first portion and a second portion configured to mate with each other to retain a distal portion of each of the plurality of segmented spines at a central spine intersection.

14. The expandable basket assembly according to claim 8, further comprising a spine retention hub disposed proximate the distal end of a tubular shaft, the spine retention hub comprising a cylindrical member including a plurality of relief lands disposed on an outer surface of the cylindrical member to allow each spine strut to be fitted into the relief land and retained therein, the spine retention hub further comprising at least one electrode disposed at a distal portion of the spine retention hub, optionally wherein the plurality of segmented spines comprises spine loops, each spine loop comprising a single unitary loop including a distal loop and two ends secured between the tubular shaft and in one of the relief lands of the spine retention hub, the distal loops overlapping within the distal retention hub, further optionally wherein the distal retention hub further comprises: two or more protrusions positioned on a first portion and/or a second portion, the plurality of segmented spines fitting within paths formed between the two or more protrusions; and two or more indentations positioned on the opposite portion of the first portion and the second portion, the indentations engaging the protrusions to clamp the first portion to the second portion, still further optionally wherein each electrode comprises a wire relief adjacent a lumen to allow for one or more wires to extend adjacent to the lumen, the lumen being disposed symmetrically about a longitudinal axis of each electrode.

15. The expandable basket assembly according to claim 8, further comprising:
a plurality of wires each electrically joined to a respective electrode,
wherein at least a portion of the wires of the plurality of the wires respectively comprises a plurality of strands and an insulative jacket circumscribing the plurality of the strands, and
wherein each strand of the plurality of strands respectively comprises an electrically conductive core material comprising a first electrical conductivity and an electrically conductive cover material comprising a second electrical conductivity less than the first electrical conductivity, the electrically conductive cover material circumscribing the electrically conductive core material.
